# EUROPEAN PATENT APPLICATION

(11) **EP 4 394 794 A1**
(43) Date of publication of application: **03.07.2024**
(21) Application number: 22217430.2
(22) Date of filing: 30.12.2022
(51) Int. Cl.: G16H 40/63, G16H 40/67, G16H 50/20, G16H 50/70, G16H 70/40, G16H 20/10

(54) **AI-BASED CUSTOMIZED MEDICAL INFORMATION PROVISION SYSTEM AND METHOD**

(71) Applicant: Lim, Myung Jae, Ansan-si 15388 (KR)
(72) Inventor: Lim, Myung Jae, Ansan-si 15388 (KR)
(74) Representative: Frenkel, Matthias Alexander

(57) **Abstract**

The present disclosure relates to a system and method for providing customized medical information based on artificial intelligence, and more particularly, a system and method in which AI technology is used to assign a certain code or number to drug specified in a prescription issued by a user and provide a list of nearby pharmacies based on user's location information, transmit prescription information to the terminal of the pharmacy selected by the patient so that prescription drugs can be prepared, provide customized drug including drug guidance video or health management video, additional information related to a disease to a user terminal and provide advertisements of medical institutions related to the customized medical information.

## Description

### TECHNICAL FIELD

The present disclosure relates to a system and method for providing customized medical information based on artificial intelligence, and more particularly, a system and method in which AI technology is used to assign a certain code or number to drug specified in a prescription issued by a user and provide a list of nearby pharmacies based on user's location information, transmit prescription information to the terminal of the pharmacy selected by the patient so that prescription drugs can be prepared, provide customized drug including drug guidance video or health management video, additional information related to a disease to a user terminal and provide advertisements of medical institutions related to the customized medical information.

### DISCUSSION OF RELATED ART

Generally, patients receive a prescription after treatment is finished, go to a pharmacy, present the prescription and purchase the prescribed drug. However, if patients have an underlying disease or a rare condition, or if they need to take multiple drugs in combination, a doctor or pharmacist will explain specific drug-taking instructions, but the contents are complicated. Hence, patients forget how to take the drug or follow the wrong instructions. In addition, it is a reality that it is difficult for patients to acquire sufficient information related to their disease or listen to explanations due to the limited doctor's consultation hours.

In addition, due to the era of super-aging, patients have a problem in that they have to take multiple drugs simultaneously as the number of drugs they need to take increases. Moreover, these drugs may differ in the timing of taking them or how they work, and there is more information to be aware of. Further, it is often difficult for elderly patients to understand simple oral drugs, but also insulin injections, inhalation devices, eye drops, and adhesive drugs, as well as auditorial explanations or written guidance when the patients have to operate and take them at home alone. Many problems arise when elderly patients acquire, understand, and store this information, as this information is explained orally by medical personnel or provided only in writing. In particular, as paper documents are provided, environmental destruction and waste of money occur.

However, producing a video of numerous drugs and health information is costly and takes a lot of time in order to address the above issues.

### SUMMARY

An object of the embodiments of the present disclosure is to provide customized medical information based on patient's prescription information.

Another object of the embodiments of the present disclosure is to provide an accurate understanding of the patient's own disease and prescription drugs to improve public health and reduce environmental waste by replacing paper documents at the same time.

Another object of the embodiments of the present disclosure is to motivate experts in the medical field to voluntarily create customized medical information contents.

An artificial intelligence-based customized medical information provision system according to a preferred embodiment of the present disclosure comprises a user terminal for transmitting prescription information received from a medical information provision server to a pharmacy terminal selected by a user and receiving customized medical information from the medical information provision server to display the customized medical information; a medical information providing server for receiving the prescription information from a hospital terminal, transmitting the prescription information to the user terminal, and transmitting the customized medical information to the user terminal; and the pharmacy terminal for displaying prescription drug order information based on the prescription information and performing a payment function, wherein the medical information provision server generates the customized medical information including at least one drug guidance video or health management video registered in the video sharing platform and additional information related to a disease based on the prescription information, registered in the video sharing platform.

As an example of the present disclosure, the medical information provision server comprises: a drug management unit for storing a combined serial number by assigning a unique number for each attribute of the drug and a unique number for each subject and additional information related to a disease; a content management unit for providing a list by serial number to at least one video providing terminal, receiving at least one drug guidance video or health management video corresponding to the serial number, and registering the video to the video sharing platform when an evaluation score is equal to or greater than a reference value; a medical information provision unit for learning a disease related to drug and health information related to the disease, calculating the degree of relevance by drug serial number for drug guidance video, health management video, and additional information related to a disease, and generating the customized medical information based on the relationship between the prescription information and the drug serial number; and a pharmacy information management unit for managing information on pre-registered pharmacies and providing a list of a plurality of nearby pharmacies based on location information of the user terminal to the user terminal, wherein the prescription information includes insurance classification, personal information, hospital information, disease classification code, doctor in charge information, drug name, single dose, number of administrations per day, total number of administration days, and usage, wherein the attributes of the drug include name, efficacy, ingredient, and content, wherein the subject of use includes gender, age group, underlying disease, and allergy, and wherein the additional information related to a disease includes any one of text, image, audio, and video.

As an example of the present disclosure, the content management unit sets the title of the drug guidance video or health management video according to certain rules based on the serial number, automatically generates subtitles for each language based on artificial intelligence, and matches advertisements related to the drug guidance video or health management video.

As an example of the present disclosure, the medical information provision server further comprises a content compensation unit for providing an incentive to a video providing terminal that has provided a drug guidance video or a health management video registered in the video sharing platform.

As an example of the present disclosure, the pharmacy terminal transmits drug guidance information according to the pharmacist's input to the user terminal.

An artificial intelligence-based customized medical information provision method according to a preferred embodiment of the present disclosure comprises: steps of transmitting, by a hospital terminal, prescription information to a medical information provision server; transmitting, by the medical information provision server, the prescription information to a user terminal; transmitting, by the user terminal, the prescription information to a pharmacy terminal selected by the user; displaying, by the pharmacy terminal, prescription drug order information based on the prescription information and performing a payment function; and transmitting, by the medical information provision server, customized medical information including at least one drug guidance video or health management video, and additional information related to a disease registered in a video sharing platform based on the prescription information to the user terminal.

As an example of the present disclosure, the method further comprises, before the step of transmitting prescription information to the medical information provision server, storing a combined serial number by assigning a unique number for each attribute of the drug and a unique number for each subject and additional information related to a disease; providing the list by serial number to at least one video providing terminal and receiving at least one drug guidance video or health management video corresponding to the serial number; registering the video to the video sharing platform when the at least one drug guidance video or health management video evaluation score is greater than or equal to a reference value; and learning a disease related to drug and health information related to the disease and calculating a degree of relevance for each drug serial number for drug guidance video, health management video, and additional information related to a disease, wherein the prescription information includes insurance classification, personal information, hospital information, disease classification code, doctor in charge information, drug name, single dose, number of administrations per day, total number of administration days, and usage, wherein the attributes of the drug include name, efficacy, ingredient, and content, wherein the subject of use includes gender, age group, underlying disease, and allergy, and wherein the additional information related to a disease includes any one of text, image, audio, and video.

As an example of the present disclosure, the step of registering the video to the video sharing platform when the at least one drug guidance video or health management video evaluation score is greater than or equal to a reference value further comprises steps of: setting a title of a drug guidance video or health management video according to a predetermined rule based on the serial number; automatically generating subtitles for each language based on artificial intelligence; and matching an advertisement related to the drug guidance video or health management video, wherein the step of transmitting, by the medical information provision server, customized medical information based on the prescription information to the user terminal further comprises step of generating the customized medical information based on the prescription information and relevancy for each drug serial number.

As an example of the present disclosure, the method further comprises step of: after the step of registering the video to the video sharing platform when the at least one drug guidance video or health management video evaluation score is greater than or equal to a reference value, providing an incentive to a video providing terminal that provided a drug guidance video or health management video registered in the video sharing platform.

As an example of the present disclosure, the step of displaying, by the pharmacy terminal, prescription drug order information based on the prescription information and performing a payment function further comprises step of transmitting drug guidance information according to the pharmacist's input to the user terminal.

According to an embodiment of the present disclosure, an expert may provide customized medical information only for a patient based on the patient's prescription information without the patient searching for necessary information.

Further, according to one embodiment of the present disclosure, a patient may be provided with an accurate understanding of the patient's own disease and prescription drugs and may actively take necessary measures for his/her own health.

Further, according to an embodiment of the present disclosure, an expert in the medical field may be induced to voluntarily produce high-quality health information contents on various drugs, diseases, and characteristics of patients.

Further, according to an embodiment of the present disclosure, it is possible to promote the development of the medical industry by identifying potential consumption demand related to a patient's disease and linking it with an advertisement of a related medical institution.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings, wherein:
FIG. 1 is a configuration diagram schematically illustrating an artificial intelligence-based customized medical information provision system according to an embodiment of the present disclosure;
FIG. 2 is a configuration diagram schematically illustrating a medical information provision server according to an embodiment of the present disclosure;
FIG. 3 is a flowchart illustrating an artificial intelligence-based customized medical information provision method according to an embodiment of the present disclosure;
FIG. 4 is a flowchart illustrating a preparation process for generating customized medical information according to an embodiment of the present disclosure; and
FIG. 5 is a flowchart illustrating a pre-processing process for a drug guidance video or health management video for registration the video to a video sharing platform according to an embodiment of the present disclosure.

### DETAILED DESCRIPTION OF EXEMPLARY EMBODIMENTS

Hereinafter, the present disclosure as described above is described in detail through the accompanying drawings and embodiments.

It should be noted that technical terms used in the present disclosure are only used to describe specific embodiments and are not intended to limit the present disclosure. Further, technical terms used in the present disclosure should be interpreted in terms commonly understood by those skilled in the art to which the present disclosure belongs, unless specifically defined otherwise in the present disclosure, and are excessively inclusive. It should not be interpreted in an excessively positive sense or in an excessively reduced sense. Further, when the technical terms used in the present disclosure are erroneous technical terms that do not accurately express the spirit of the present disclosure, they should be replaced with technical terms that those skilled in the art can correctly understand. Further, general terms should be interpreted as defined in the dictionary or according to context and should not be interpreted in an excessively reduced sense.

Further, singular expressions used in the present disclosure include plural expressions unless the context clearly indicates. Terms such as "consisting of" or "comprising" used in the present disclosure should not be construed as necessarily including all of the various components or steps described in the invention, and it should be construed that some components or steps among them may not be included, or additional components or steps may be further included.

Further, terms including ordinal numbers such as "first" and "second" used in the present disclosure may be used to describe components, but components should not be limited by the terms. Terms are used only to distinguish one component from another. For example, a first element may be termed a second element, and similarly, a second element may be termed a first element, without departing from the scope of the present disclosure. Hereinafter, preferred embodiments according to the present disclosure are described in detail with reference to the accompanying drawings. However, regardless of reference numerals, the same or similar components are given the same reference numerals, and overlapping descriptions thereof are excluded.

Further, in describing the present disclosure, if it is determined that a detailed description of a related known technology may obscure the gist of the present disclosure, the detailed description will be excluded. Further, it should be noted that the accompanying drawings are only for easily understanding the spirit of the present disclosure and should not be construed as limiting the spirit of the present disclosure by the accompanying drawings.

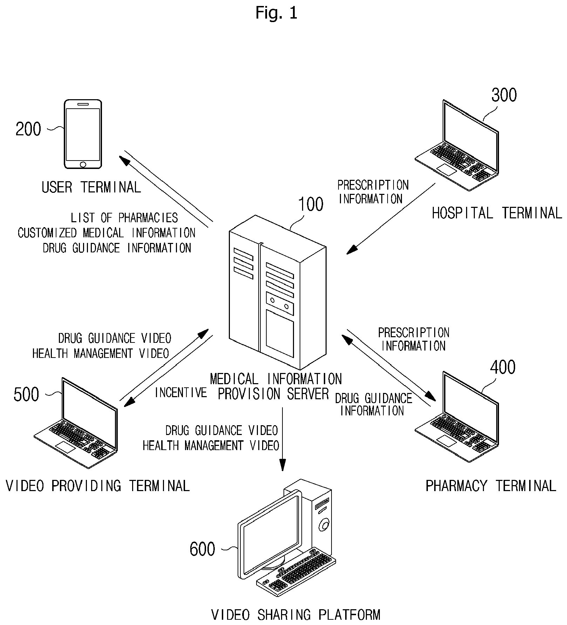

FIG. 1 is a configuration diagram schematically illustrating an artificial intelligence (AI)-based customized medical information provision system according to an embodiment of the present disclosure.

Referring to FIG. 1, the AI-based customized medical information provision system may comprise a medical information provision server 100, a user terminal 200, a hospital terminal 300, a pharmacy terminal 400, a video providing terminal 500, and a video sharing platform 600.

Each component of FIG. 1 is generally connected through a network. For example, the user terminal 200 may be connected to the medical information provision server 100, the pharmacy terminal 400, and the video sharing platform 600 through a network. The medical information provision server 100 may be connected to the user terminal 200, the hospital terminal 300, the pharmacy terminal 400, the video providing terminal 500, the video sharing platform 600, etc., through a network.

The network refers to a connection structure capable of exchanging information between nodes such as a plurality of terminals and servers, and examples of such networks include a local area network (LAN) and a wide area network (WAN), the Internet (WWW: World Wide Web), wired and wireless data communications networks, telephone networks, and wired and wireless television communications networks. Examples of wireless data communication networks include 3G, 4G, 5G, 3rd generation partnership project (3GPP), 5th generation partnership project (5GPP), long-term evolution (LTE), world interoperability for microwave access (WiMAX), Wi-Fi, Internet, local area network (LAN), wireless local area network (wireless LAN), wide area network (WAN), personal area network (PAN), radio frequency (RF), Bluetooth network, near-field communication (NFC) networks, satellite broadcasting networks, analog broadcasting networks, digital multimedia broadcasting (DMB) networks, etc., but are not limited thereto.

The user terminal 200 may transmit the prescription information received from the medical information providing server 100 to the pharmacy terminal 400 selected by the user. It may receive customized medical information from the medical information provision server 100 and display the customized medical information.

The prescription information is information input by a doctor to issue a prescription. This includes various information such as insurance classification, personal information, hospital information, disease classification code, the doctor in charge information, drug name, single dose, number of administrations per day, the total number of administration days, and usage. The prescription information input by a doctor in the hospital terminal 300 may be automatically transmitted to the medical information provision server 100.

According to an embodiment, the user terminal 200 may receive the prescription information through a customized medical information application provided by the medical information provision server 100.

Further, when a list of a plurality of nearby pharmacies based on the user's location information identified through a built-in GPS or mobile communication network is displayed in the customized medical information application of the user terminal 200, the user terminal 200 may transmit the prescription information according to the user's selection to the pharmacy terminal 400 corresponding to the selected pharmacy. Further, the prescription information may be obtained through optical character recognition (OCR) of an image of a prescription provided by a hospital by the user, or through QR code recognition provided in a prescription. This way, prescription information may be obtained through prescription information input from the hospital terminal 300 and OCR or QR code recognition of the user terminal 200.

According to an embodiment, when a screen displayed on a blood glucose meter or a blood pressure monitor is photographed through a camera (not shown) of the user terminal 200 and gender and age are input, if gender and age are input, the captured screen, gender, and age are analyzed to automatically provide in video with high relevance, thereby checking user's health condition and reviewing products or services related to user's disease through advertisements provided along with the video. Further, when a specific word is entered on the search screen of the customized medical information application, a list of videos related to the word is provided, and specialized video information may be provided to the user by selecting it.

The medical information provision server 100 may receive prescription information from the hospital terminal 300, transmit the prescription information to the user terminal 200 and transmit the customized medical information to the user terminal 200. More specifically, the medical information provision server 100 generates medical information, including at least one drug guidance video, health management image, or additional information related to a disease registered in the video-sharing platform 600 based on the prescription information. The medical information provision server 100 may provide a customized medical information application to the user terminal 200 and provide customized medical information through the customized medical information application installed in the user terminal 200.

The medical information provision server 100 provides prescription information, customized medical information, and a list of a plurality of nearby pharmacies based on location information of the user terminal 200 through a customized medical information application installed on the user terminal 200.

According to an embodiment, the medical information provision server 100 provides various customized medical information such as drug guidance video, health management video, and additional information related to a disease through a customized medical information application. However, it provides customized advertisements related to each piece of information may be of interest to the user.

The hospital terminal 300 may be a terminal into which prescription information is input by a doctor. The hospital terminal 300 may be configured to automatically transmit the prescription information to the medical information provision server 100 as soon as the prescription information is input.

According to an embodiment, the prescription information may be obtained through optical character recognition (OCR) of an image taken by the user terminal 200 of a prescription provided by a user from a hospital. Further, the prescription information may be obtained through QR code recognition by providing a QR code to the prescription. This way, prescription information may be directly obtained from the user terminal 200 and transmitted to the pharmacy terminal 400.

According to an embodiment, the hospital terminal 300 may request registration of advertisements for hospital information and medical products provided by the hospital to the medical information provision server 100. For example, it is possible to request registration of advertisements for health-functional foods, supplements, treatment-related medical devices, etc., helpful for the user's disease to the medical information provision server 100.

The pharmacy terminal 400 may be a terminal displaying prescription drug order information based on the prescription information received from the medical information provision server 100 and performing a payment function.

According to an embodiment, the pharmacy terminal 400 may request registration of advertisements for pharmacy information and various products and the like to the medical information provision server 100. For example, like the hospital terminal 300, information on health-functional foods or supplements suitable for the type of disease of the user identified through prescription information is produced as an advertisement, and the pharmacy terminal 400 may request registration of advertisements to the medical information provision server 100.

In this way, the medical information provision server 100 may receive advertisement requests from various institutions related to medical care, such as the hospital terminal 300, pharmacy terminal 400, insurance company, financial company, etc., and store them in a database (not shown), and provide advertisements highly related to a disease of a specific patient among stored advertisements to the user terminal 200. Here, the degree of relevance, which is a criterion for matching, may be based on keywords.

According to an embodiment, the pharmacy terminal 400 may transmit drug guidance information input based on the prescription information to the user terminal 200. In this case, the drug guidance information may be simply inputted by the pharmacist through the pharmacy terminal 400, such as used for prescription drugs. For example, drug guidance information in the form of voice or text from the pharmacist regarding 30 minutes after a meal, 30 minutes before a meal, 3 times a day, a list of foods to be careful of during the intake period, possible side effects and countermeasures may be provided.

In this way, prescription information is transmitted to the selected pharmacy terminal based on the location of the user terminal 200 in advance so that without the user arriving at the pharmacy after treatment, presenting the prescription, selecting the pharmacy and waiting for prescription drug to be prepared, the desired pharmacy is selected and prescription information is transmitted immediately, time to pick up the prescription may be saved upon arrival at the pharmacy. Further, drug guidance information input from the pharmacist is received to check again at any time about usage or precautions, which helps to take medicine normally according to the usage.

The video providing terminal 500 may receive a combined serial number by assigning a unique number for each attribute of the drug and a unique number for each subject from the medical information provision server 100 and produce at least one drug guidance video or health management video corresponding to the serial number. The video providing terminal 500 may transmit at least one drug guidance video or health management video to the medical information provision server 100. To explain this in detail, Tylenol^{™} is given a unique number for each attribute such as 6 digits 112201 through antipyretic analgesic-11, acetaminophen-22, and 500 mg-01. Further, if the content is changed to 650mg, although the efficacy and the same ingredient, the last two digits may be designated as 02. Further, additional unique numbers for gender and age are assigned to the above number so that a unique number for each attribute of drug and a unique number for each subject of drug may be given to store the combined serial number. For example, there are 112201-MO, 112201-FY, 112201-Y, and 112201-0 in which serial numbers such as M=male, F=female, Y=young, and O=old may be assigned to each drug. Further, additional unique numbers may be assigned for underlying diseases, allergies, and the like.

According to one embodiment, preferably, students of medical school, pharmacy school, dental school, etc., which may be regarded as experts in the medical field, may provide drug guidance video or health management video through the video providing terminal 500 but are limited thereto. Those who can produce medical information-related videos may also provide drug guidance video or health management video. In this way, high-quality content may be supplied through cloud sourcing for the serial number list provided by the medical information provision unit. The serial number list may be displayed on the video providing the terminal 500 with the serial number and names, efficacy, ingredients, and contents, which are attributes of drugs corresponding to the serial numbers. In addition, video standard information including video time, quality, format, etc. may be additionally provided to secure video consistency. The obtained drug guidance video and health management video are evaluated to calculate an evaluation score. When the evaluation score is above a reference value, it is registered on a video-sharing platform, and an incentive may be paid to the video provider. For example, the evaluation of a drug guidance video or health management video may be performed by qualified holders of a specialist or higher in each department pre-registered in the medical information provision server 100, and a video with a score of 25 points or more on a scale of 30 points may be determined as registration. Evaluation standards may include accuracy of the information, professionalism, delivery power, and the like. Qualified holders of specialists in each department or higher can score points on a scale of 10 points for each evaluation standard. After the evaluation of a certain number of qualified holders is completed, the average of the evaluation scores is derived, and the video whose average score exceeds 25 points may be determined as registration.

According to one embodiment, the drug guidance video or health management video may be set to be viewed only by qualified holders of each department specialist or higher registered in the medical information provision server 100, and only the video for which registration has been decided may be registered in the video sharing platform 600.

The video-sharing platform 600 may be a social media platform such as YouTube, Facebook, and Instagram. The video-sharing platform 600 may share various media types, including text, image, audio, and video.

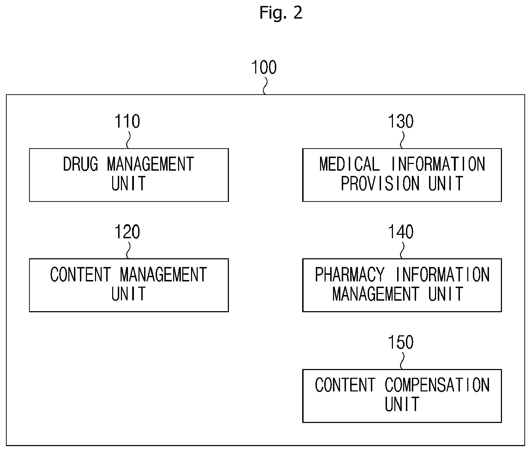

According to an embodiment, a drug guidance video or health management video determined to be registered by the medical information provision server 100 may be registered in the video-sharing platform 600. The detailed descriptions are described later with reference to FIGS. 2, 3, and 4.

FIG. 2 is a configuration diagram schematically illustrating a medical information provision server 100 according to an embodiment of the present disclosure.

Referring to FIG. 2, the medical information provision server 100 comprises a drug management unit 110, a content management unit 120, a medical information provision unit 130, a pharmacy information management unit 140, and a content compensation unit 150.

The drug management unit 110 may store a combined serial number by assigning a unique number for each attribute of drug and a unique number for each subject of drug and additional information related to a disease. For example, TylenolTM is given a unique number for each attribute, such as 6 digits 112201 through antipyretic analgesic-11, acetaminophen-22, and 500 mg-01. Further, if the content is changed to 650mg, although the efficacy and the same ingredient, the last two digits may be designated as 02. Further, additional unique numbers for gender and age are assigned to the above number so that a unique number for each attribute of drug and a unique number for each subject of drug may be given to store the combined serial number. For example, there are 112201-MO, 112201-FY, 112201-Y, and 112201-0 in which serial numbers such as M=male, F=female, Y=young, and O=old may be assigned to each drug. In the case of age, for example, generation may be classified based on the age of 40, Y (young) may be assigned to those younger than 40 years old, and O (elderly) may be assigned to those over 60 years old.

According to one embodiment, additional unique numbers may be assigned for underlying diseases, allergies, and the like. AL may be assigned for allergies, and numbers may be assigned according to the type of allergy so that it may be configured to have a unique number such that AL01 may be assigned for drug allergies and AL02 may be assigned for food allergies. In the case of an underlying disease, the corresponding disease classification code may be assigned as a unique number. Therefore, the serial number for each drug is based on the combination of the unique number for each attribute of the drug and the unique number for each subject to be taken. However, the serial number may be created by giving an additional unique number for the underlying disease or allergy. Accordingly, when information such as an underlying disease or allergy is input by the user, the accuracy of customized medical information to be provided to the user may be further increased.

The content management unit 120 may provide the list by serial number to at least one video providing terminal 500, receive at least one drug guidance video or health management video corresponding to the serial number, and register the video to the video sharing platform 600 when the evaluation score is above the reference value. Specifically, the serial number list may be displayed on the video providing terminal 500 with the serial number and the name, efficacy, ingredient, and content of drug corresponding to the serial number. Further, video standard information including video time, quality, format, etc., may be additionally provided to secure video consistency. The obtained drug guidance video and health management video are evaluated, and an evaluation score is calculated. When the evaluation score is higher than the reference value, it is registered on the video-sharing platform, and incentives may be paid to the video provider. For example, the evaluation of a drug guidance video or health management video may be performed by qualified holders of a specialist or higher in each department pre-registered in the medical information provision server 100, and a video with a score of 25 points or more on a scale of 30 points may be determined as registration. Evaluation standards may include accuracy of the information, professionalism, delivery power, and the like. Qualified holders of specialists in each department or higher can score points on a scale of 10 points for each evaluation standard. After the evaluation of a certain number of qualified holders is completed, the average of the evaluation scores is derived, and the video whose average score exceeds 25 points may be determined as registration.

The content management unit 120 sets the title of the drug guidance video or health management video according to a predetermined rule based on the serial number, automatically creates subtitles for each language based on artificial intelligence, and automatically matches advertisements related to the drug guidance video or health management video.

More specifically, setting the title of a drug guidance video or health management video according to a certain rule based on the serial number is as follows. For example, Tylenol is assigned a drug serial number of 112201-MO, which is a combination of a 6-digit unique number for antipyretic analgesic-11, acetaminophen-22, and 500 mg-01 and a unique number for each subject. In this case, since there is information such as antipyretic analgesic, acetaminophen, 500 mg, male, elderly, titles such as "Elderly male's cold medicine taking guidance" or "Elderly male's cold prevention and treatment" are given to the video received with the serial number for each drug. Further, when a unique number for an underlying disease or allergy is added, titles such as Elderly male with diabetes's cold medicine taking guidance" or "Elderly male with diabetes's cold prevention and treatment" may be assigned.

Subtitles for each language may be generated through automatic translation after first converting the voice in the video into text using the AI-based speech-to-text method. The AI-based speech-to-text method may be implemented using open speech processing APIs such as Google Cloud Speech API, IBM Watson Speech to Text, Microsoft Azure Bing Speech API, and Amazon Transcribe. Automatic translation for each language may be implemented using commercial artificial intelligence automatic translation API services such as Google Translation API, Kakao Translation API, and Papago Translation API.

Further, an advertisement related to a cold of a diabetic elderly male may be matched based on the serial number for each drug, and the corresponding advertisement may be provided at the bottom of the video or added before or after the video.

The medical information provision unit 130 may learn drug-related diseases and health information related to the diseases, calculate the degree of relevance by drug serial number for drug guidance video, health management video, and additional information related to a disease and generate the customized medical information based on the degree of relevance between prescription information and each drug serial number.

Specifically, only one prescription drug may be provided for a simple disease such as a cold, but multiple drugs may be prescribed according to cold symptoms or a doctor's prescription, and various types of drugs may be prescribed according to the type of disease. Therefore, big data on prescriptions are collected, and preprocessing is performed on the big data on prescriptions first. The information included in the prescription includes insurance classification, personal information, hospital information, disease classification code, the doctor in charge information, drug name (efficacy and ingredient), single dose (content), number of administrations per day, total number of days of administration, usage, etc. Among them, personal information, disease classification code, drug name, single dose, number of administrations per day, the total number of days of the administration, usage, etc. may be labeled, and the relevance between disease classification code and personal information and at least one drug may be learned. Further, among personal information, sensitive information other than age and gender may be excluded. Further, age may be transformed into age group. For example, 32 years old is set as 30 years old group, and 56 years old is set as 50 years old group to prevent data fragmentation. Based on the preprocessed data, the degree of relevance for each drug serial number may be calculated according to the disease classification code, age, and gender through machine learning, and the accuracy of the machine learning algorithm may be corrected through a verification process.

The pharmacy information management unit 140 may manage information on registered pharmacies and provide a list of a plurality of nearby pharmacies to the user terminal 200 based on location information of the user terminal 200. Based on the the location information of the user terminal 200, a nearby pharmacy may be displayed on a map through a customized medical information application, or a list of pharmacies may be displayed in order of proximity.

The content compensation unit 150 may provide an incentive to the video-providing terminal 500 that has provided the drug guidance video or health management video registered in the video-sharing platform 600. Incentives may be provided in various forms, such as cash payment through an account, points, or coupons. Therefore, video providers may have the motivation to produce higher-quality content.

The attributes of the drug include efficacy, ingredients, and content, and the subject of the drug includes gender, age group, underlying disease, and allergy. The additional information related to a disease may be any one of text, image, audio, and video. For example, for diabetes, news related to causes, symptoms, treatment, prevention, diet and lifestyle, new drugs, or new treatments of diabetes may be provided in the form of text, image, audio, or video. If necessary, the latest information may be appropriately processed and provided in the form of card news.

According to an embodiment, the medical information provision server 100 may store the prescription information in a private blockchain. Prescription information is patients' sensitive information, so the risk of hacking can be prevented by storing it in the blockchain. To this end, it is possible to provide users with control over individual prescription information based on DID identity authentication (decentralized identity). If there is a user's consent, the medical information provision server 100 may use the prescription information and provide various customized medical information.

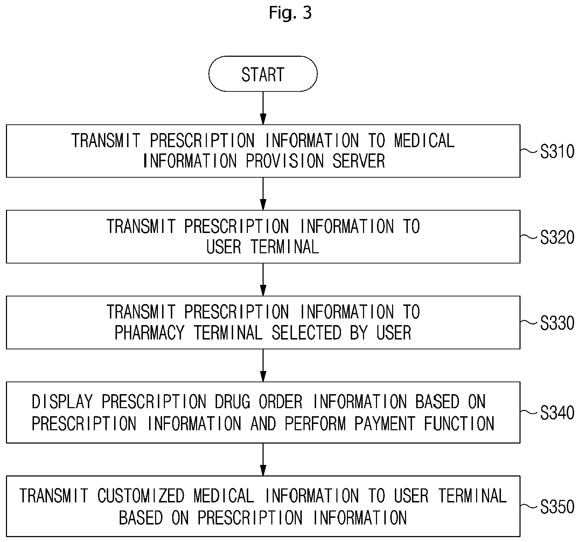

FIG. 3 is a flowchart illustrating an artificial intelligence-based customized medical information provision method according to an embodiment of the present disclosure.

Referring to FIG. 3, the method may comprise steps S310 of transmitting, by a hospital terminal 300, prescription information to a medical information provision server 100; S320 of transmitting, by the medical information provision server 100, the prescription information to a user terminal 200; S330 of transmitting, by the user terminal 200, the prescription information to a pharmacy terminal 400 selected by the user; S340 of displaying, by the pharmacy terminal 400, prescription drug order information based on the prescription information and performing a payment function; and S350 of transmitting, by the medical information provision server 100, customized medical information including at least one drug guidance video or health management video, and additional information related to a disease registered in a video sharing platform 600 based on the prescription information to the user terminal 200.

S310 of transmitting, by a hospital terminal 300, prescription information to a medical information provision server 100 may be configured to transmit automatically. When the doctor inputs the prescription information into the hospital terminal 300 to issue a prescription, the prescription information is sent to the medical information provision server 100 simultaneously. The prescription information includes insurance classification, personal information, hospital information, disease classification code, the doctor in charge information, drug name, single dose, number of administrations per day, the total number of days of the administration, and usage.

In S320 of transmitting, by the medical information provision server 100, the prescription information is sent to a user terminal 200. When the medical information provision server 100 receives the prescription information, it automatically transmits a push notification notifying that the user terminal 200 has received the prescription information, and the prescription information may be received and displayed through the user terminal 200. According to an embodiment, the user terminal 200 may receive the prescription information through a customized medical information application provided by the medical information provision server 100.

In S330 of transmitting, by the user terminal 200, the prescription information to a pharmacy terminal 400 selected by the user, the user terminal 200 may display nearby pharmacies on a map or display a list of pharmacies in order of local distance based on location information determined based on GPS or a mobile communication network. The prescription information is transmitted to the pharmacy terminal 400 of the selected pharmacy among them based on the user input.

In S340 of displaying, by the pharmacy terminal 400, prescription drug order information based on the prescription information and performing a payment function, the pharmacy terminal 400 receives the prescription information. It displays prescription drug order information, and the pharmacist may confirm receipt of the prescription information through a notification function or the like. Further, a message notifying reception of prescription information may be transmitted to a pharmacist's portable terminal (not shown) connected to the pharmacy terminal 400 through a communication network.

In S350 of transmitting, by the medical information provision server 100, customized medical information including at least one drug guidance video or health management video, and additional information related to a disease registered in a video sharing platform 600 based on the prescription information to the user terminal 200, the drug guidance video or health management video may be directly embedded and played in a customized medical information application. Further, ink information on the drug guidance video and health management video may be provided through a customized medical information application, messenger, or text message according to the selection by the user terminal 200. The link information of the drug guidance video or health management video indicates the address information of the drug guidance video or health management video registered in the video sharing platform 600.

S350 of transmitting, by the medical information provision server 100, customized medical information based on the prescription information to the user terminal 200 may further comprise the step of generating the customized medical information based on the relevance between the prescription information and each drug serial number (not shown). In other words, information such as disease classification code, age, drug name, single dose, number of administrations per day, the total number of days of the administration, and usage are extracted from prescription information, and the drug serial number most closely related to this is selected. A drug guidance video, health management video, or additional information related to a disease corresponding to the drug serial number may be generated as customized medical information. The customized medical information generated by the medical information provision server 100 may be provided through a push alarm or a medical information-providing application. Further, the customized medical information may be checked within the medical information-providing application. Further, it may be directly moved to the video-sharing platform 600 and checked according to the user's selection.

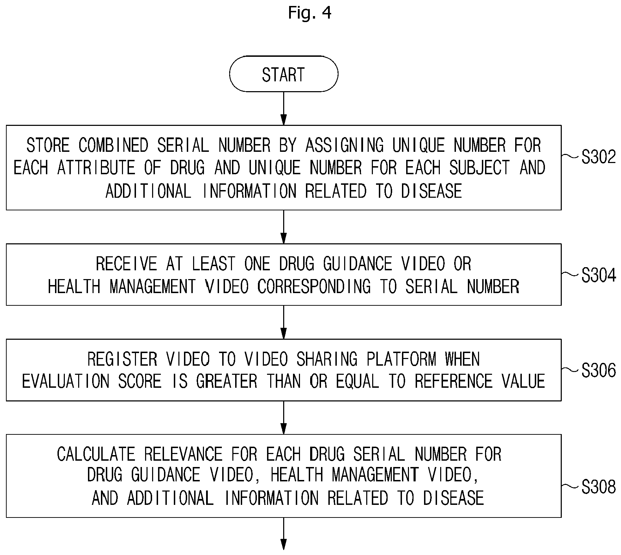

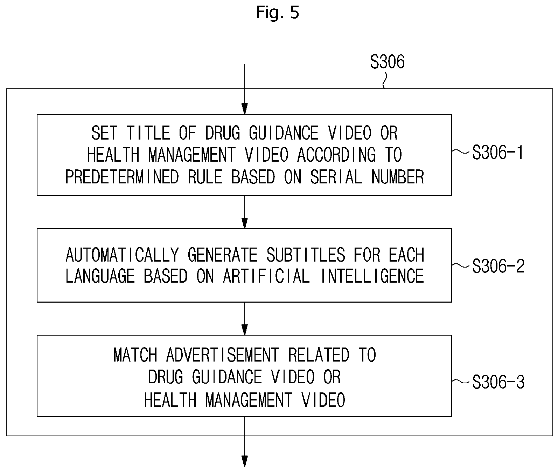

FIG. 4 is a flowchart illustrating a preparation process for generating customized medical information according to an embodiment of the present disclosure, and FIG. 5 is a flowchart illustrating a preprocessing process for a drug guidance video or health management video for registration the video to a video sharing platform 600 according to an embodiment of the present disclosure.

Referring to FIG. 4, the method may comprise, before S310 of transmitting prescription information to the medical information provision server 100, S302 of storing a combined serial number by assigning a unique number for each attribute of the drug and a unique number for each subject and additional information related to a disease; S304 of providing the list by serial number to at least one video providing terminal 500 and receiving at least one drug guidance video or health management video corresponding to the serial number; S306 of registering the video to the video sharing platform 600 when the at least one drug guidance video or health management video evaluation score is greater than or equal to a reference value; and S308 of learning a disease related to drug and health information related to the disease and calculating a degree of relevance for each drug serial number for drug guidance video, health management video, and additional information related to a disease.

According to one embodiment, the prescription information may include insurance classification, personal information, hospital information, disease classification code, the doctor in charge information, drug name, single dose, number of administrations per day, the total number of administration days, and usage, the attributes of the drug may include name, efficacy, ingredient, and content, the subject of use includes gender, age group, underlying disease, and allergy, and the additional information related to a disease may include any one of text, image, audio, and video.

In S302, storing a combined serial number by assigning a unique number for each attribute of the drug and a unique number for each subject and additional information related to a disease. For example, of a unique number for each drug attribute, TylenolTM may be configured as 6 digits 112201 through antipyretic analgesic-11, acetaminophen-22, and 500 mg-01. Further, if the content is changed to 650 mg although the efficacy and the same ingredient, the last two digits may be designated as 02. Further, additional unique numbers for gender and age are assigned to the above number so that a unique number for each attribute of drug and a unique number for each subject of drug may be given to store the combined serial number. For example, there are 112201-MO, 112201-FY, 112201-Y, and 112201-O in which serial numbers such as M=male, F=female, Y=young, and O=old may be assigned to each drug. In the case of age, for example, generation may be classified based on the age of 40, Y (young) may be assigned to those younger than 40 years old, and O (elderly) may be assigned to those over 60 years old.

According to one embodiment, additional unique numbers may be assigned for underlying diseases, allergies, and the like. AL may be assigned for allergies, and numbers may be assigned according to the type of allergy so that it may be configured to have a unique number such that AL01 may be assigned for drug allergies, and AL02 may be assigned for food allergies. In the case of an underlying disease, the corresponding disease classification code may be assigned as a unique number. Therefore, the serial number for each drug is based on the combination of the unique number for each attribute of the drug and the unique number for each subject to be taken. However, the serial number may be created by giving an additional unique number for the underlying disease or allergy. Accordingly, when information such as an underlying disease or allergy is input by the user, the accuracy of customized medical information to be provided to the user may be further increased.

In S304 of providing the list by serial number to at least one video providing terminal 500 and receiving at least one drug guidance video or health management video corresponding to the serial number, the serial number list may be displayed on the video providing terminal 500 together with the serial number and corresponding attributes of the drug, such as name, efficacy, ingredient, and content. Further, video standard information, including video time, quality, format, etc., may be additionally provided to secure video consistency. The video providing terminal 500 may receive a list of combined serial numbers of a unique number for each property of drug and a unique number for each subject of drug assigned by the medical information provision server 100 and create and transmit at least one drug guidance video or health management video corresponding to the serial number to the medical information provision server 100. Preferably, students of medical school, pharmacy school, dental school, etc., which may be regarded as experts in the medical field, may provide drug guidance video or health management video through the video providing terminal 500 but are limited thereto. Those who can produce medical information-related videos may also provide a drug guidance video or health management video. This way, high-quality content can be supplied and supplied through the cloud-sourcing format for the serial number list provided by the medical information provision server.

Referring to FIGS. 4 and 5, S306 of registering the video to the video sharing platform 600 when at least one drug guidance video or health management video evaluation score is greater than or equal to a reference value may further comprise S306-1 of setting a title of a drug guidance video or health management video according to a predetermined rule based on the serial number; S306-2 of automatically generating subtitles for each language based on artificial intelligence; and S306-3 of matching an advertisement related to the drug guidance video or health management video.

In S306 of registering the video to the video sharing platform 600 when at least one drug guidance video or health management video evaluation score is greater than or equal to a reference, the drug guidance video or health management video received from the video providing terminal 500 is evaluated, and the evaluation score is calculated. The video may be registered on the video-sharing platform if the evaluation score exceeds the reference value. For example, the evaluation of a drug guidance video or health management video may be performed by qualified holders of a specialist or higher in each department pre-registered in the medical information provision server 100, and a video with a score of 25 points or more on a scale of 30 points may be determined as registration. Evaluation standards may include accuracy of the information, professionalism, delivery power, and the like. Qualified holders of specialists in each department or higher can score points on a scale of 10 points for each evaluation standard. After the evaluation of a certain number of qualified holders is completed, the average of the evaluation scores is derived, and the video whose average score exceeds 25 points may be determined as registration.

In S306-1, setting the title of a drug guidance video or health management video according to a certain rule based on the serial number is as follows. For example, Tylenol is assigned a drug serial number of 112201-MO, which is a combination of a 6-digit unique number for antipyretic analgesic-11, acetaminophen-22, and 500 mg-01 and a unique number for each subject. In this case, since there is information such as antipyretic analgesic, acetaminophen, 500 mg, male, elderly, titles such as "Elderly male's cold medicine taking guidance" or "Elderly male's cold prevention and treatment" are given to the video received with the serial number for each drug. Further, when a unique number for an underlying disease or allergy is added, titles such as Elderly male with diabetes's cold medicine taking guidance" or "Elderly male with diabetes's cold prevention and treatment" may be assigned.

For example, Tylenol is assigned a drug serial number of 112201-MO, which is a combination of a 6-digit unique number for antipyretic analgesic-11, acetaminophen-22, and 500 mg-01 and a unique number for each subject. In this case, since there is information such as antipyretic analgesic, acetaminophen, 500 mg, male, elderly, titles such as "Elderly male's cold medicine taking guidance" or "Elderly male's cold prevention and treatment" are given to the video received with the serial number for each drug. Further, when a unique number for an underlying disease or allergy is added, titles such as Elderly male with diabetes's cold medicine taking guidance" or "Elderly male with diabetes's cold prevention and treatment" may be assigned.

In S306-2, subtitles for each language may be generated through automatic translation after first converting the voice in the video into text using the AI-based speech-to-text method. The AI-based speech-to-text method may be implemented using open speech processing APIs such as Google Cloud Speech API, IBM Watson Speech to Text, Microsoft Azure Bing Speech API, and Amazon Transcribe. Automatic translation for each language may be implemented using commercial artificial intelligence automatic translation API services such as Google Translation API, Kakao Translation API, and Papago Translation API. Text may be generated from the audio of the received video by applying speech to text (STT) method, and subtitles for each language may be generated by applying artificial intelligence automatic translation technology to the text. Therefore, subtitle data for each language may be added to the video. Accordingly, although the user is not fluent in Korean, it is possible to obtain information more easily through subtitles for each language provided in the video.

In S306-3, an advertisement related to a cold of a diabetic elderly male may be matched based on the serial number for each drug, and the corresponding advertisement may be provided at the bottom of the video or added before or after the video. Among advertisements registered from various organizations or institutions, an advertisement with high relevance may be matched based on the serial number for each drug.

For example, advertisements selected through keyword-based advertisement matching may be provided to videos. The advertisement may be provided in text or image at the bottom of the video or before or after the video.

In S308 of learning a disease related to drug and health information related to the disease and calculating a degree of relevance for each drug serial number for the drug guidance video, health management video, and additional information related to a disease, only one prescription drug may be provided for a simple disease such as a cold, but multiple drugs may be prescribed according to cold symptoms or a doctor's prescription, and various types of drugs may be prescribed according to the type of disease. Therefore, big data on prescriptions are collected, and preprocessing is performed on the big data on prescriptions first. The information included in the prescription includes insurance classification, personal information, hospital information, disease classification code, the doctor in charge information, drug name (efficacy and ingredient), single dose (content), number of administrations per day, the total number of days of administration, usage, etc. Among them, personal information, disease classification code, drug name, single dose, number of administrations per day, the total number of days of the administration, usage, etc., may be labeled, and the relevance between disease classification code and personal information and at least one drug may be learned. Further, among personal information, sensitive information other than age and gender may be excluded. In the case of age, for example, generation may be classified based on the age of 40, Y (young) may be assigned to those younger than 40 years old, and O (elderly) may be assigned to those over 60 years old. The degree of relevance for each drug serial number may be calculated according to the disease classification code, age, and gender through machine learning based on the preprocessed data, and the accuracy of the machine learning algorithm may be corrected through a verification process.

According to one embodiment, the method may further comprise, after S306 of registering the video to the video sharing platform 600 when at least one drug guidance video or health management video evaluation score is greater than or equal to a reference, providing an incentive to a video providing terminal 500 that provided a drug guidance video, or a health management video registered in the video sharing platform 600. For example, in S306, incentives may be provided in various forms, such as cash payment through an account, points, or coupons to the video provider of the video providing terminal 500 that provided a drug guidance video or health management video having an evaluation score higher than the reference value in various forms such as cash payment through an account, points, coupons, etc. Therefore, video providers may produce higher-quality content.

According to one embodiment, S340 of displaying, by the pharmacy terminal 400, prescription drug order information based on the prescription information and performing a payment function may further comprise the step of transmitting drug guidance information according to the pharmacist's input to the user terminal 200 (not shown). The drug guidance information may be information such as directions for prescription drugs which the pharmacist input through the pharmacy terminal 400. For example, drug guidance information such as 30 minutes after a meal or 30 minutes before a meal, 3 times a day, a list of foods to be careful of, possible side effects, and countermeasures during the intake period may be provided in the form of a pharmacist's voice or text. Further, the drug guidance information may be provided by converting it into text form through voice recognition of the pharmacist's voice or video.

As described above, in the artificial intelligence-based customized medical information providing system and method according to an embodiment of the present disclosure, when a user registered as a member finishes treatment at a hospital, the user may immediately receive prescription information through a medical information provision application. When the user selects a pharmacy from a list of nearby pharmacies based on the location information of the user terminal, the prescription information may be transmitted to the pharmacy terminal of the selected pharmacy, and the pharmacy may immediately receive it. Further, customized medical information generated based on drug guidance information prepared by a pharmacist, and the prescription information may be provided through a medical information provision application. Thus, from the user's point of view, the user may receive a variety of information such as prescription drug purchases, disease-related information, drug guidance video, health management video, additional information related to a disease, and advertisements for various products or services related to his or her disease after treatment and re-check the same if necessary. Therefore, these results in an increase in the convenience of health care and an improvement in the quality of life.

Further, service providers may obtain various medical information through the collection of prescription information to provide higher quality customized medical information, and hospitals, pharmacies, and other related organizations registered in the service may be linked to create synergistic effects.

In the above, preferred embodiments according to the present disclosure have been shown and described. However, the present disclosure is not limited to the above-described embodiments, and various modifications can be made by anyone having ordinary knowledge in the technical field to which the present disclosure belongs without departing from the gist of the present disclosure appended within the scope of the claims.

## Claims

1. A system for providing customized information based on artificial intelligence (AI), comprising:
a user terminal for transmitting prescription information received from a medical information provision server to a pharmacy terminal selected by a user and receiving customized medical information from the medical information provision server to display the customized medical information;
a medical information providing server for receiving the prescription information from a hospital terminal, transmitting the prescription information to the user terminal, and transmitting the customized medical information to the user terminal; and
the pharmacy terminal for displaying prescription drug order information based on the prescription information and performing a payment function;
wherein the medical information provision server generates the customized medical information including at least one drug guidance video or health management video registered in the video sharing platform and additional information related to a disease based on the prescription information, registered in the video sharing platform.

2. The system of claim 1, wherein the medical information provision server comprises:
a drug management unit for storing a combined serial number by assigning a unique number for each attribute of the drug and a unique number for each subject and additional information related to a disease;
a content management unit for providing a list by serial number to at least one video providing terminal, receiving at least one drug guidance video or health management video corresponding to the serial number, and registering the video to the video sharing platform when an evaluation score is equal to or greater than a reference value;
a medical information provision unit for learning a disease related to drug and health information related to the disease, calculating the degree of relevance by drug serial number for drug guidance video, health management video, and additional information related to a disease, and generating the customized medical information based on the relationship between the prescription information and the drug serial number; and
a pharmacy information management unit for managing information on pre-registered pharmacies and providing a list of a plurality of nearby pharmacies based on location information of the user terminal to the user terminal;
wherein the prescription information includes insurance classification, personal information, hospital information, disease classification code, doctor in charge information, drug name, single dose, number of administrations per day, total number of administration days, and usage,
wherein the attributes of the drug include name, efficacy, ingredient, and content,
wherein the subject of use includes gender, age group, underlying disease, and allergy, and
wherein the additional information related to a disease includes any one of text, image, audio, and video.

3. The system of claim 2, wherein the content management unit sets the title of the drug guidance video or health management video according to certain rules based on the serial number, automatically generates subtitles for each language based on artificial intelligence, and matches advertisements related to the drug guidance video or health management video.

4. The system of claim 2 or 3, wherein the medical information provision server further comprises a content compensation unit for providing an incentive to a video providing terminal that has provided a drug guidance video or a health management video registered in the video sharing platform.

5. The system of any one of claims 1 to 4, wherein the pharmacy terminal transmits drug guidance information according to the pharmacist's input to the user terminal.

6. A method for providing customized information based on AI, the method comprising: steps of
transmitting, by a hospital terminal, prescription information to a medical information provision server;
transmitting, by the medical information provision server, the prescription information to a user terminal;
transmitting, by the user terminal, the prescription information to a pharmacy terminal selected by the user;
displaying, by the pharmacy terminal, prescription drug order information based on the prescription information and performing a payment function; and
transmitting, by the medical information provision server, customized medical information including at least one drug guidance video or health management video, and additional information related to a disease registered in a video sharing platform based on the prescription information to the user terminal.

7. The method of claim 6, further comprising, before the step of transmitting prescription information to the medical information provision server,
storing a combined serial number by assigning a unique number for each attribute of the drug and a unique number for each subject and additional information related to a disease;
providing the list by serial number to at least one video providing terminal and receiving at least one drug guidance video or health management video corresponding to the serial number;
registering the video to the video sharing platform when the at least one drug guidance video or health management video evaluation score is greater than or equal to a reference value; and
learning a disease related to drug and health information related to the disease and calculating a degree of relevance for each drug serial number for drug guidance video, health management video, and additional information related to a disease;
wherein the prescription information includes insurance classification, personal information, hospital information, disease classification code, doctor in charge information, drug name, single dose, number of administrations per day, total number of administration days, and usage,
wherein the attributes of the drug include name, efficacy, ingredient, and content,
wherein the subject of use includes gender, age group, underlying disease, and allergy, and
wherein the additional information related to a disease includes any one of text, image, audio, and video.

8. The method of claim 7, wherein the step of registering the video to the video sharing platform when the at least one drug guidance video or health management video evaluation score is greater than or equal to a reference value further comprises steps of:
setting a title of a drug guidance video or health management video according to a predetermined rule based on the serial number;
automatically generating subtitles for each language based on artificial intelligence; and
matching an advertisement related to the drug guidance video or health management video;
wherein the step of transmitting, by the medical information provision server, customized medical information based on the prescription information to the user terminal further comprises step of generating the customized medical information based on the prescription information and relevancy for each drug serial number.

9. The method of any one of claims 6 to 8, further comprising step of: after the step of registering the video to the video sharing platform when the at least one drug guidance video or health management video evaluation score is greater than or equal to a reference value,
providing an incentive to a video providing terminal that provided a drug guidance video or health management video registered in the video sharing platform.

10. The method of any one of claims 6 to 9, wherein the step of displaying, by the pharmacy terminal, prescription drug order information based on the prescription information and performing a payment function further comprises step of transmitting drug guidance information according to the pharmacist's input to the user terminal.
